(19) Europäisches Patentamt
European Patent Office
Office européen des brevets

(11) Veröffentlichungsnummer: **0 412 467 A1**

(12) **EUROPÄISCHE PATENTANMELDUNG**

(21) Anmeldenummer: **90115010.2**

(51) Int. Cl.5: **C12M 1/40**

(22) Anmeldetag: **04.08.90**

(30) Priorität: **11.08.89 DE 3926609**

(43) Veröffentlichungstag der Anmeldung:
**13.02.91 Patentblatt 91/07**

(84) Benannte Vertragsstaaten:
**AT BE CH DE DK ES FR GB GR IT LI LU NL SE**

(71) Anmelder: **Solvay Enzymes GmbH & Co. KG**
**Grosse Drakenburger Strasse 103**
**D-3070 Nienburg/Weser(DE)**

(72) Erfinder: **Geyer, Hans-Ulrich**
**Kramerstrasse 8**
**D-3000 Hannover 1(DE)**

(74) Vertreter: **Lauer, Dieter, Dr.**
**c/o Kali-Chemie AG Postfach 220**
**Hans-Böckler-Allee 20**
**D-3000 Hannover 1(DE)**

(54) **Neues Verfahren zur kontinuierlichen Durchführung enzymatischer Umsetzungen mit immobilisierten Biokatalysatoren.**

(57) Beschrieben wird ein vollkontinuierliches Verfahren zur Umsetzung einer Substratlösung an einem immobilisierten Biokatalysator. Hierzu wird die Substratlösung am Katalysator mit Enzym-Aktivität in einem Rohrreaktor umgesetzt, wobei der Katalysator derart entlang des von der Substratlösung durchströmten Rohrreaktors wandert, daß längs dieses Rohrreaktors ein zeitlich im wesentlichen konstantes Aktivitätsprofil aufrecht erhalten wird. Dieses Verfahren ermöglicht die Einhaltung einer weitgehend gleichbleibenden Raumgeschwindigkeit der Substratlösung bzw. einen gleichmäßigen Produktstrom mit im wesentlichen gleichbleibendem Umwandlungsgrad.

EP 0 412 467 A1

# NEUES VERFAHREN ZUR KONTINUIERLICHEN DURCHFÜHRUNG ENZYMATISCHER UMSETZUNGEN MIT IMMOBILISIERTEN BIOKATALYSATOREN

Die Erfindung bezieht sich auf ein neues Verfahren zur kontinuierlichen Durchführung enzymatischer Umsetzungen, bei dem eine Lösung des herzustellenden Produktes erhalten wird, indem eine Substratlösung an einem immobilisierten Biokatalysator mit Enzymaktivität für die Substrat-Produkt-Umwandlung in einem Rohrreaktor umgesetzt wird.

Quasikontinuierlich ausführbare industrielle Verfahren zur enzymatischen Umwandlung von Substraten, bei denen die Substratlösung einen Reaktor durchströmt, der einen immobilisierten Biokatalysator enthält, sind bereits bekannt. Bei diesen bekannten Verfahren bestehen jedoch Probleme, da die Enzymaktivität des Biokatalysators in der Regel mit fortschreitender Betriebszeit des Reaktors mehr oder weniger schnell abnimmt.

Es muß daher zum Ausgleich des Aktivitätsverlustes die Einwirkzeit des immobilisierten Biokatalysators auf die Substratlösung erhöht werden, indem man die Raumgeschwindigkeit der Substratlösung (Volumen Substratlösung pro vom Katalysator in Anspruch genommenes Reaktorvolumen pro Stunde = v/vh) vermindert und dadurch an die sinkende Enzymaktivität anpaßt.

Die Raumgeschwindigkeit der Substratlösung kann aus technischer und wirtschaftlicher Sicht jedoch nicht beliebig weit vermindert werden, sondern sie wird durch praxisgerechte Mindestgeschwindigkeiten zu unteren Werten hin begrenzt. Dieses bedeutet, daß nicht die gesamte, in den Reaktor eingebrachte Enzymaktivität ausgenutzt werden kann. Beim Erreichen der Mindestraumgeschwindigkeit ist der eingesetzte immobilisierte Biokatalysator keinesfalls vollständig aufgebraucht (d.h. bereits ohne Aktivität), sondern er weist in der Regel eine an sich noch beachtliche Restaktivität von etwa 20 % der Anfangsaktivität auf. Diese nach den bekannten Verfahren nicht nutzbare Restaktivität wird in der Regel verworfen. Darüber hinaus ist die vorgenannte Verfahrensweise aus verfahrenstechnischer Sicht auch deshalb nachteilig, weil sie einen ungleichmäßigen Produktstrom bedingt, zu dessen Ausgleich eine Vielzahl von Reaktoren erforderlich ist.

Es bestand daher die Aufgabe, ein kontinuierliches Verfahren zur enzymatischen Umwandlung von Substraten mit immobilisierten Biokatalysatoren (im folgenden auch Katalysator mit Enzymaktivität oder kurz Katalysator genannt) zur Verfügung zu stellen, welches mit einer im wesentlichen gleichbleibenden Raumgeschwindigkeit der Substratlösung arbeitet und somit einen im wesentlichen gleichmäßigen Strom einer das enzymatische Umwandlungsprodukt enthaltenden Lösung mit im wesentlichen gleichbleibendem Umwandlungsgrad ermöglicht. Die Aufgabe wird gelöst durch das erfindungsgemäße Verfahren.

Die Erfindung betrifft ein Verfahren zur kontinuierlichen Herstellung einer Lösung eines enzymatisch herstellbaren Produktes durch Umsetzung einer Substratlösung an einem immobilisierten Biokatalysator mit Enzymaktivität für die Substrat-Produkt-Umwandlung in einem Rohrreaktor, bei dem man den immobilisierten Biokatalysator längs des von der Substratlösung durchströmten Rohrreaktors derart bewegt, daß längs dieses Rohrreaktors ein zeitlich im wesentlichen konstantes Aktivitätsprofil aufrecht erhalten wird.

Während bei den Verfahren gemäß Stand der Technik der Katalysator als nicht bewegte, stationäre Phase im Reaktor vorliegt und nur der Substrat/Produkt-Strom als mobile Phase den Reaktor durchströmt, wandert beim Verfahren der Erfindung auch der immobilisierte Biokatalysator mit Enzymaktivität durch den Reaktor. Das erfindungsgemäße Verfahren arbeitet also mit zwei mobilen Phasen, die relativ zueinander durch den Reaktor bewegt werden. Der Substrat/Produkt-Strom kann hierbei prinzipiell sowohl im Gleichstrom als auch im Gegenstrom zum mobilen Katalysator mit Enzymaktivität geführt werden. Der Transport der beiden mobilen Phasen kann durch an sich bekannte Maßnahmen bewirkt werden. Bezogen auf die mobile Festphase des Katalysators kann der Transport z.B. durch Einwirkung der Schwerkraft, eines hydrodynamischen Druckes oder durch konventionelle mechanische Transportelemente bewerkstelligt werden.

Das Verfahren der Erfindung unterscheidet sich von denen gemäß Stand der Technik durch ein zeitlich im wesentlichen konstantes Aktivitätsprofil längs des Rohrreaktors. Unter Aktivitätsprofil wird hier der räumliche Verlauf der Aktivität des immobilisierten Biokatalysators längs des Reaktors jeweils zu einem bestimmten Zeitpunkt der Durchführung des Verfahrens verstanden. Bei den Verfahren gemäß Stand der Technik verändert sich das Aktivitätsprofil aufgrund der sich an jedem Ort des Reaktors mit zunehmender Verfahrensdauer vermindernden Enzymaktivität. Es liegt bei den bekannten Verfahren ein zeitlich nicht konstantes Aktivitätsprofil und eine Abnahme der Gesamtenzymaktivität im Reaktor vor, die eine Anpassung der Raumgeschwindigkeit der Substratlösung durch ständiges Vermindern derselben erzwingt.

Die Enzymaktivität der einzelnen Katalysatorteilchen nimmt zwar auch beim erfindungsgemäßen Verfahren ab, doch werden die Katalysatorteilchen entsprechend ihrer Aktivitätsabnahme durch den Reaktor bewegt, so daß sie jeweils aus einer Zone des Reaktors mit der höheren Aktivität in eine benachbarte Zone

mit niedrigerer Aktivität befördert werden. Hierdurch wird in jeder Zone eine definierte, stationäre Enzymaktivität eingestellt und somit entlang des Reaktors ein zeitlich im wesentlichen konstantes Aktivitätsprofil aufrecht erhalten. Die Gesamtenzymaktivität des Reaktors bleibt im wesentlichen konstant und das Verfahren kann demgemäß mit einer weitgehend gleichbleibenden Raumgeschwindigkeit der Substratlösung durchgeführt werden.

In einer Variante des erfindungsgemäßen Verfahrens wird das Aktivitätsprofil aufrecht erhalten, indem man ständig frischen immobilisierten Biokatalysator zu- und inaktiven Katalysator abführt. Hierzu wird der Rohrreaktor an einem Ende ständig mit frischem, immobilisiertem Biokatalysator befüllt, welcher dann mit gegebener Geschwindigkeit durch den Rohrreaktor wandert, und am anderen Ende des Reaktors wird schließlich die äquivalente Volumenmenge an inaktivem immobilisierten Biokatalysator entfernt. Zufuhr und Abfuhr erfolgen im wesentlichen synchron und können durch an sich bekannte Maßnahmen bewerkstelligt werden.

In einer anderen günstigen Variante des erfindungsgemäßen Verfahrens wird das Aktivitätsprofil aufrecht erhalten, indem man intervallweise frischen immobilisierten Biokatalysator zu- und inaktiven Katalysator abführt. Hierzu wird z.B. der Rohrreaktor zu Beginn eines Zeitintervalls an einem Ende mit einer definierten Volumenmenge an frischem, immobilisiertem Biokatalysator befüllt, welcher dann intervallweise den Rohrreaktors durchwandert, und am anderen Ende des Reaktors wird schließlich die äquivalente Volumenmenge an inaktivem Katalysator ent fernt. Zufuhr und Abfuhr können dabei im wesentlichen zum gleichen Zeitpunkt erfolgen und durch an sich bekannte Maßnahmen bewerkstelligt werden. Es ist aber auch möglich, die intervallweise Zufuhr und Abfuhr zu unterschiedlichen Zeitpunkten durchzuführen. Zu Beginn eines oder einiger aufeinanderfolgender Zeitintervalle wird dann zunächst nur frischer immobilisierter Biokatalysator zugeführt, aber noch kein inaktiver Katalysator abgeführt. Die Abfuhr des inaktiven Katalysators wird erst zu Beginn eines späteren Zeitintervalls vorgenommen, wobei dann eine entsprechend größere Volumenmenge an inaktivem Katalysator aus dem Reaktor abgezogen wird. Die Zu- und Abfuhr von Katalysator zu unterschiedlichen Zeitpunkten zeigt keine Auswirkungen auf die Form und die Konstanz des Aktivitätsprofils, mit der Einschränkung, daß es sich in dem Maße, wie sich der inaktive Katalysatorpfropfen am Ende des Reaktors aufbaut oder dieser entfernt wird, nur insgesamt längs des Reaktors entsprechend verschiebt. Dieses asynchrone Vorgehen hat verfahrenstechnisch den Vorteil, daß ggf. weniger häufig Entnahmen von inaktivem Katalysator aus dem Reaktor in den Betriebsablauf einzuplanen sind und der Füllstand des Reaktors den Betriebserfordernissen besser angepaßt werden kann. In einer Abwandlung der intervallweisen Durchführung des Verfahrens kann man auch so vorgehen, daß nur der inaktive Katalysator intervallweise abgeführt wird, frischer immobilisierter Biokatalysator dagegen ständig zugeführt wird. Die Abfuhr des inaktiven Katalysators kann dabei zu gegebenen Zeitpunkten erfolgen, spätestens jedoch, wenn jeweils der maximale Füllstand des Reaktors erreicht ist.

Beim vorstehend beschriebenen Intervallverfahren sinkt in jeder Zone des Reaktors die Enzymaktivität innerhalb der festgelegten Zeitintervalle zunächst von einem Anfangswert zu Beginn des Intervalls auf einen niedrigeren Endwert am Ende des Intervalls ab. Da jedoch zu Beginn eines jeden neuen Zeitintervalls Katalysatorteilchen entsprechend ihrer Aktivitätsabnahme aus jeweils einer Zone des Reaktors mit einer höheren Aktivität in eine benachbarte Zone mit niedrigerer Aktivität befördert werden, wird in jeder Zone eine definierte, im Mittel quasistationäre Enzymaktivität eingestellt. Durch diese mittleren Enzymaktivitäten wird entlang des Reaktors dann das zeitlich im wesentlichen konstante Aktivitätsprofil definiert. Das Intervallverfahren kann demgemäß mit einer mit Mittel gleichbleibenden Raumgeschwindigkeit der Substratlösung, die an die im Mittel im wesentlichen konstante Gesamtenzymaktivität des Reaktors angepaßt ist, durchgeführt werden. Die Raumgeschwindigkeit nimmt zu Beginn eines Intervalles von einem Anfangswert auf einen niedrigeren Endwert ab und wird zu Beginn des nächsten Intervalls wieder auf den Anfangswert angehoben.

Prinzipiell können der immobilisierte Biokatalysator und die Substratlösung sowohl im Gegenstrom als auch im Gleichstrom zueinander durch den Reaktor bewegt werden. In einer bevorzugten Variante des erfindungsgemäßen Verfahrens führt man den Katalysator im Gleichstrom zur Substratlösung durch den Rohrreaktor.

Grundsätzlich kann man den Rohrreaktor beliebig anordnen, in einer besonders bevorzugten Variante des erfindungsgemäßen Verfahrens wird der immobilisierte Biokatalysator jedoch entlang eines vertikal angeordneten Rohrreaktors bewegt. Der Transport des Katalysators kann entweder durch Wirkung der Gravitation oder z.B. durch Transportelemente bewirkt werden, die die Katalysatorteilchen über die Höhe des Katalysatorbettes vom oberen Ende der Reaktorsäule langsam zum unteren Ende der Reaktorsäule transportieren, wo sie schließlich die mechanischen Austragungselemente erreichen und aus der Reaktorsäule entfernt werden. Bevorzugt geschieht der Transport der Katalysatorteilchen durch Einwirkung der Gravitation. Ein derart betriebener Reaktor hat dann ein Katalysatorbett, an dessen oberem Ende frischer

immobilisierter Biokatalysator mit in etwa ständig 100 % Anfangsaktivität vorliegt. In den darunterliegenden Katalysatorbettschichten nimmt die Aktivität bis zum unteren Ende des Katalysatorbettes ständig ab. Am Reaktorausgang liegt im wesentlichen nur noch Katalysator ohne Aktivität vor.

Führt man das Verfahren der Erfindung in einem vertikal angeordneten Reaktor durch, so wird zu Beginn des Verfahrens das zeitlich im wesentlichen konstante Aktivitätsprofil aufgebaut, indem man in einer Anfangsphase die Umsetzung zunächst mit einer Grundschüttung aus immobilisiertem Biokatalysator in der Reaktorsäule in an sich bekannter Weise beginnt und dann, sobald die Enzymaktivität dieser Grundschüttung auf einen unteren Grenzwert abgesunken ist, ständig oder intervallweise frischen immobilisierten Biokatalysator bis zum Erreichen einer gewünschten oder der maximalen Höhe der Schüttung zugibt. In der Praxis geht man dabei so vor, daß man am oberen Ende der Reaktorsäule frischen immobilisierten Biokatalysator vorzugsweise im Gleichstrom in den Substratstrom einspeist oder direkt in den Reaktor einfüllt. Der zugeführte Katalysator mit Enzymaktivität sinkt dann auf das Anfangsbett ab, wobei das Katalysatorbett allmählich erhöht wird. Im Hinblick auf eine hohe Kapazitätsausnutzung des Reaktors ist es günstig, mit der Zufuhr von frischem immobilisierten Biokatalysator bereits zu beginnen, sobald die Aktivität auf einen Wert unter 95 %, vorzugsweise auf einen Wert zwischen 70 bis 90 % der Anfangsaktivität abgesunken ist. Die Menge des neu zugeführten immobilisierten Biokatalysators wird dabei so bemessen, daß die darin enthaltene Aktivitätsmenge derjenigen äquivalent ist, die inaktiviert wurde. Durch die Kompensation des Inaktivierungsverlustes durch frischen immobilisierten Biokatalysator wird die bei Verfahren gemäß Stand der Technik erforderliche Absenkung der Raumgeschwindigkeit der Substratlösung beim Verfahren der Erfindung weitgehend vermieden. Die Reaktorsäule wird also mit im wesentlichen konstanter Raumgeschwindigkeit und gleichbleibendem Umwandlungsgrad gefahren. Sobald aufgrund der Katalysatorzufuhr die Schüt tung des Katalysators eine vorgegebene oder die maximale Füllhöhe der Reaktorsäule erreicht, ist die Anfangsphase mit dem Aufbau des Aktivitätsprofils in der Reaktorsäule abgeschlossen. Das Verfahren geht nun in den erfindungsgemäß kontinuierlichen Betrieb über. Unter Beibehaltung der ständigen oder intervallweisen Zufuhr von frischem immobilisierten Biokatalysator wird nun auch mit der ständigen oder intervallweisen Entfernung von bereits inaktivem Katalysator am unteren Ende der Reaktorsäule begonnen.

Der Reaktor kann auch ohne Ausnutzung der vollen Kapazität, z.B. bei schwächerer Produktnachfrage, gefahren werden. Dann wird in der Anfangsphase mit der Zufuhr von frischem immobilisierten Biokatalysator erst dann begonnen, wenn die Aktivität des Reaktors auf niedrigere Werte der Anfangsaktivität, als oben für volle Kapazitätsauslastung angegeben abgesunken ist. Hierbei können bis zu einer vom jeweiligen Biokatalysator abhängigen, technisch und wirtschaftlich noch sinnvollen Untergrenze beliebige Prozentwerte der Anfangsaktivität im Reaktor eingestellt und in Analogie zu den oben beschriebenen Verfahrensweisen, durch ständige bzw. intervallweise Zu- und Abfuhr von immobilisiertem Biokatalysator aufrecht erhalten werden.

Durch das erfindungsgemäße Verfahren ist es somit möglich, den Arbeitspunkt, d.h. das Aktivitätsniveau, des Reaktors dem Produktbedarf in weiten Grenzen exakt anzupassen.

Es versteht sich von selbst, daß die Einstellung eines gegebenen Aktivitätsniveaus nicht nur während der vorstehend beschriebenen Anfangsphasen zum Aufbau des Aktivitätsprofils erfolgen kann, sondern auch zu jedem Zeitpunkt der erfindungsgemäß kontinuierlichen Betriebsphase des Reaktors. So kann durch entsprechende Verzögerung oder Unterbrechung der Zufuhr von frischem immobilisierten Biokatalysator von hohen auf niedrigere Aktivitätsniveaus übergegangen werden oder im umgekehrten Falle durch zusätzliche Zufuhr von frischem immobilisierten Biokatalysator auch von niedrigen auf höhere Aktivitätsniveaus umgestellt werden.

Das vom immobilisierten Biokatalysator umzuwandelnde Substrat wird im erfindungsgemäßen Verfahren in Form einer klaren Lösung, wie sie im allgemeinen auch bei konventionellen Verfahren Verwendung findet, in den Reaktor eingebracht. Als Lösungsmittel für das Substrat können Wasser und an sich alle mit dem jeweiligen Biokatalysator verträglichen, ggf. sogar aktivierenden und/oder stabilisierenden organischen Lösungsmittel verwendet werden. Geeignete organische Lösungsmittel sind z.B. geradkettige oder verzweigte aliphatische C1- bis C10-Alkohole, vorzugsweise C1- bis C5-Alkohole wie Methanol, Ethanol, Isopropanol, Butanole oder Pentanole, und andere organische Lösungsmittel wie Ether, Dioxan, Dimethylsulfoxid, Dimethylformamid, Pyridin etc. Besonders geeignet sind Wasser und solche organischen Lösungsmittel die mit Wasser, vollständig oder wenigstens teilweise mischbar sind. Es versteht sich von selbst, daß auch Gemische der geeigneten Lösungsmittel eingesetzt werden können.

Im erfindungsgemäßen Verfahren können an sich beliebige immobilisierte Biokatalysatoren eingesetzt werden. Unter immobilisierten Biokatalysatoren werden hier sowohl immobilisierte Enzyme als auch immobilisierte Mikroorganismen verstanden. In besonderem Maße eignet sich das Verfahren jedoch für solche Enzyme, die ein flüssiges, klares Substrat bzw. eine klare Lösung eines Substrates in einem geeigneten

Lösungsmittel bzw. Lösungsmittelgemisch verlangen. Die Vorteile des erfindungsgemäßen Verfahrens kommen dabei insbesondere bei solchen Enzymen zum Tragen, die zu beschleunigter Desaktivierung neigen und demgemäß bei konventionellen Verfahren in nachteiliger Weise nur eine beschränkte Nutzungsdauer der Reaktorfüllung zulassen. Beispiele für die im erfindungsgemäßen Verfahren verwendbaren Enzyme sind Cellulasen, Glucoamylasen, alpha-Amylasen, Glucosidasen, Glucoseoxidasen, Glucoseisomerasen, Lactasen, Invertasen, Lipasen, Esterasen, Acylasen, Proteasen etc. Werden demgegenüber Mikroorganismen als Biokatalysatoren eingesetzt, so können diese insbesondere Hefen, Pilze oder Bakterien sein, die die vorgenannten Enzyme besitzen und somit die entsprechenden enzymatischen Reaktionen ausführen können.

In einer speziellen Ausgestaltung des erfindungsgemäßen Verfahrens wird beispielsweise immobilisierte Glucoseisomerase als Katalysator mit Enzymaktivität zur Umwandlung von Glucose zu Fructose eingesetzt, indem man eine Glucose-haltige Substratlösung durch die weiter oben beschriebene Reaktorsäule, d.h. über das Wanderbett mit gerichtet bewegten Enzymträgerteilchen und mit zeitlich im wesentlichen konstantem Aktivitätsprofil leitet.

Je nach Art der eingesetzten Glucoseisomerase, werden bei der Durchführung des Verfahrens die im Stand der Technik an sich bekannten Reaktionsbedingungen wie Temperatur, pH-Wert, Raumgeschwindigkeit oder sonstige Parameter der Substratlösung usw. eingehalten und sofern erwünscht, können darüber hinaus auch weitere im Stand der Technik bekannte Maßnahmen wie beispielsweise Zugabe bestimmter Ionen oder Cofaktoren zur Substratlösung, Zugabe einer stabilisierenden Menge eines Antioxidants zur Substratlösung oder andere an sich bekannte Vorbehandlungsmaßnahmen der Substratlösung ergriffen werden. Konkrete Bedingungen lassen sich diesbezüglich insbesondere den deutschen Patentschriften DE 34 05 035 und DE 31 48 603 entnehmen.

Zur Durchführung des Verfahrens der Erfindung mit Glucoseisomerase wird für ein vorgegebenes Aktivitätsniveau des Reaktors die Raumgeschwindigkeit der Substratlösung zweckmäßigerweise so auf die Geschwindigkeit des Katalysators abgestimmt, daß sich die Substratlösung und der Katalysator mit einer relativen Geschwindigkeit zu einander bewegen, die der Raumgeschwindigkeit der Substratlösung bei einem vergleichbaren Aktivitätsniveau eines Reaktors im konventionellen Verfahren entspricht. Diese relative Raumgeschwindigkeit ist dabei so eingestellt, daß die Ablaufende Produktlösung, bezogen auf Trockensubstanz, z.B. einen Gehalt an Fructose von 42,5 ± 0,5 Gew.-% aufweist. Je nach Glucosegehalt des Substrates muß dazu ein Isomerisierungsgrad zwischen 43 und 47 % eingestellt werden.

In dieser Ausgestaltung des erfindungsgemäßen Verfahrens kann an sich jede an einen anorganischen oder organischen Träger adsorptiv oder kovalent gebundene Glucoseisomerase verwendet werden. Bevorzugt werden solche an sich bekannten Katalysatoren mit Glucoseisomerase-Aktivität eingesetzt, bei denen die Glucoseisomerase auf porösem Siliciumdioxid oder porösem Aluminiumoxid immobilisiert vorliegt. Geeignete Katalysatoren dieser Art sind z.B. solche, die gemäß der DE 27 26 188 durch Immobilisierung von Glucoseisomerase gewonnen werden können und die z.B. auch in bekannten Verfahren gemäß DE 31 48 603 und 34 05 035 Verwendung finden.

Die nach dem erfindungsgemäßen Verfahren erhaltene Glucose-Fructose-Lösung kann in an sich bekannter Weise ihrer endgültigen Verwendung zugeführt werden. Beispielsweise kann es empfehlenswert oder erwünscht sein, während des Verfahrens eingebrachte aber z.B. geschmacksbeeinträchtigende ionische Komponenten durch Kationen- und Anionenaustausch aus dem Produkt zu entfernen. Ggf. kann die Lösung noch zum Sirup (Isosirup oder Isoglucose) eingeengt oder zu Lösungen mit geringeren oder höheren Fructosegehalten als 42 Gew.-% verarbeitet werden.

Der grundsätzliche Vorteil des erfindungsgemäßen Verfahrens besteht darin, daß die Aktivität des immobilisierten Biokatalysators vollständig ausgenutzt werden kann. Bei den Verfahren im Stand der Technik bleibt dagegen eine Restaktivität von ca. 20 % der Anfangsaktivität ungenutzt.

Das erfindungsgemäße Verfahren ermöglicht darüber hinaus eine vollkontinuierliche Umwandlung von Substrat bei im wesentlichen konstanter Raumgeschwindigkeit des Substrat/Produkt-Stromes und somit eine konstante Produktivität des Reaktors pro Zeiteinheit. Es ist dabei sehr vorteilhaft, daß die konstante Raumgeschwindigkeit auch eine konstante kurze Verweilzeit des empfindlichen Substrat/Produkt-Gemisches im Reaktor ermöglicht. Die Bildung unerwünschter Nebenprodukte wie z.B. Hydroxymethylfurfural (HMF), Acetaldehyd oder Psicose sowie eine unerwünschte Farbe der Substrat/Produktlösung wird dadurch vermieden.

Der Reaktor läßt sich in einer vorteilhaften Variante mit konstantem Durchsatz auf einem hohen Aktivitäts- und Raumgeschwindigkeitsniveau fahren, wodurch die Sicherheit des Systems gegenüber mikrobieller Kontamination beträchtlich erhöht wird. Weiterhin kann der Reaktor bei jedem technisch und wirtschaftlich sinnvollen Niveau der Aktivität des Biokatalysators gefahren werden, wobei ein Wechsel zwischen verschiedenen Niveaus der Aktivität ohne weiteres möglich ist. Wird dem Reaktor häufig genug

frischer immobilisierter Biokatalysator zugeführt, kann ein vorgegebenes Aktivitätsniveau in engen Grenzen aufrecht erhalten werden. Beispielsweise ist es bei einer Inaktivierungsrate von ca. 3 % in 100 Stunden bereits ausreichend, wenn dem Reaktor etwa zweimal pro Woche frischer immobilisierter Biokatalysator zugeführt wird.

Darüber hinaus bestehen auch erhebliche verfahrenstechnische und apparative Vorteile. So ermöglicht das erfindungsgemäße Verfahren ein vollkontinuierliches Be- und Entladen des Reaktors während der Umsetzung. Gegenüber dem Stand der Technik entfallen Stillstände zum Be- und Entchargen. Die Zahl der benötigten Reaktoren (im Stand der Technik üblicherweise 6 bis 10) kann sogar bis auf einen Reaktor reduziert werden. Damit werden Investitions-und Unterhaltungskosten außerordentlich gesenkt und die betriebliche Kontrollanalytik sehr vereinfacht.

Die nachfolgenden Beispiele dienen der Erläuterung der Erfindung, ohne sie jedoch in ihrem Umfang zu beschränken.

## Beispiele

### 1. Allgemeine Bedingungen und Materialien

1.1 Als Versuchsreaktor wurde eine temperierbare Glassäule mit einem Innendurchmesser von 0,7 cm vertikal in abwärts gerichteter Fließrichtung betrieben.

1.2 Zur Durchführung des Verfahrens wurde ein immobilisierter Biokatalysator mit Glucoseisomerase-Aktivität vom Typ Optisweet H (Miles Kali-Chemie GmbH & Co. KG) mit einer Anfangsaktivität, die (als Raumgeschwindigkeit ausgedrückt) 7,4 v/vh (60 °C) betrug, eingesetzt.

1.3 Das eingesetzte Substrat hatte folgende Zusammensetzung:

| | |
|---|---|
| Glucose: | 45 Gew.-% |
| Magnesium: | 120 ppm |
| $SO_2$: | 600 ppm |
| pH-Wert: | 7,5 |

Magnesium wurde in Form von Magnesiumsulfat und $SO_2$ in Form von Natriumsulfit zugegeben.

1.4 Um das Verfahren der Erfindung in einem vertretbaren Zeitrahmen demonstrieren zu können, wurde nicht bei der an sich üblichen Verfahrenstemperatur von 60 °C, sondern bei erhöhter Temperatur von 75 °C gearbeitet. Die Anfangsaktivität des Katalysators bei 75 °C ist 2,5-fach höher als bei 60 °C, die hier bewußt angestrebte hohe Inaktivierungsrate bei 75 °C ist ca. 10-fach größer als bei 60 °C. Die nachfolgenden Meßergebnisse konnten so in einem vertretbaren Zeitraum erhalten werden.

### 2. Durchführung des Verfahrens

2 ml des Katalysators mit Glucoseisomerase-Aktivität und den unter 1.2 beschriebenen Eigenschaften wurden als Grundschüttung in den Reaktor eingebracht (Füllhöhe 5,2 cm). Durch diesen Reaktor wurde mit einer Dosierpumpe die vortemperierte (75 °C) Substratlösung geleitet und der Durchsatz dabei so eingestellt und ggf. nachreguliert, daß der Isomerisierungsgrad über die gesamte Betriebszeit konstant 46,5 % betrug (Arbeiten bei konstantem Isomerisierungsgrad); Anfangsgeschwindigkeit = 18,5 v/vh. Nachfolgend wurde nach vorgegebenen Zeitintervallen ("Intervallverfahren") jeweils eine bestimmte Menge an frischem Katalysator mit Glucoseisomerase-Aktivität auf die im Reaktor vorgelegte Schüttung bis zum Erreichen der maximalen Füllhöhe des Reaktcrs zugegeben (Ende der Aufbauphase und Beginn des vollkontinuierlichen Betriebes). Die Katalysatorzugaben erfolgten jeweils bei Werten zwischen 75 % bis 50 % für die Restaktivität, wobei die zugeführten Mengen jeweils etwa das Anfangsniveau (100 %) der Enzymaktivität wiederherstellten. (Bei einem größer ausgelegten Reaktor ist eine wesentlich kleinere Abstufung der zugeführten Katalysatormenge technisch möglich. Bei den Reaktordimensionen des vorliegenden Beispiels konnten aus Gründen der Genauigkeit keine feineren Abstufungen gewählt werden, da dann die jeweils zuzuführende Katalysatormenge zu gering ausgefallen wäre.) Die zugegebene Katalysatormenge wurde in ml abgemessen und zur Erhöhung der Genauigkeit aus der Zunahme der Aktivität exakt in Gramm berechnet. Insgesamt wurde während des Verlaufs des Versuches achtmal frischer immobilisierter

Biokatalysator nachgefüllt.

Die Entnahme von inaktivem Katalysator am Reaktorauslauf erfolgte während der vollkontinuierlichen Betriebsphase insgesamt zweimal nach jeweils 4 Nachfüllungen zu den Zeitpunkten 410 h (Entnahme 1 g) und 700 h (Entnahme 1,483 g).

Im Anschluß an die letzte Katalysatorentnahme nach einer Betriebszeit von 700 h wurde die Zugabe von frischem immobilisierten Biokatalysator eingestellt und der Reaktor bis zum Erreichen einer Restaktivität von 20 % weiter betrieben. Diese letzte Phase entspricht dem Herunterfahren des Betriebsniveaus der Enzymaktivität im Reaktor. Wird während dieser Phase bei einem Aktivitätsniveau oberhalb 20 % wieder mit der Zugabe von frischem immobilisierten Biokatalysator begonnen, besteht die Möglichkeit, den Reaktor auf einem frei wählbaren Betriebsniveau der Aktivität oberhalb von 20 % weiterzufahren.

Die Ergebnisse dieses Beispiels sind als Verlauf der Enzymaktivität im Reaktor in % der Anfangsaktivität über die Betriebszeit angegeben. Die Betriebsdaten und -ergebnisse sind im einzelnen in der Tabelle 1 und in den Figuren 1 und 2 aufgeführt.

3. Betriebsdaten und Ergebnisse

3.1 Tabelle 1

Aktivitätsverlauf in Abhängigkeit von Betriebszeit und zugeführtem bzw. entnommenem Katalysator sowie Durchsatz und Produktivität (ermittelt per Integration über die Nachfüllabschnitte).

| Betriebszeitraum (h) | Aktivitäts-Spanne (%) | Durchsatz in g HFS-TS | | **Katalysator** | | | |
|---|---|---|---|---|---|---|---|
| | | g/Δt | g/Σt | Nachfüllmenge und -zeit | | Reaktor-Inhalt (g) | Entnahme (g) |
| | | | | g | t(h) | | |
| $t_0$ | 100 | - | - | - | - | 1 | - |
| 0- 73 | 100 -73,1 | 1254,0 | 1254 | 0,2538 | 73 | 1,2538 | - |
| 73-171 | 101,5-61,5 | 1566,9 | 2820,9 | 0,4488 | 171 | 1,7026 | - |
| 171-265 | 112,2-60,9 | 1581,1 | 4402,0 | 0,4184 | 265 | 2,1210 | - |
| 265-339 | 104,6-65,4 | 1236,3 | 5638,3 | 0,3623 | 339 | 2,4833 | - |
| 339-410 | 103,5-71,5 | 1213,0 | 6851,3 | 0,3468 | 410 | 2,8301 | - |
| 410 | | | | | | 1,8301 | 1,00 [1]) |
| 410-506 | 106,7-58,0 | 1503,6 | 8354,9 | 0,4613 | 506 | 2,2914 | - |
| 506-578 | 102,5-66,0 | 1195,5 | 9550,4 | 0,3346 | 578 | 2,6260 | - |
| 578-700 | 95,6-47,5 | 1748,4 | 11298,8 | 0,5808 | 700 | 3,2068 | - |
| 700 | | | | | | 1,7238 | 1,483 [2]) |
| 700-940 | 105,6-17,8 | 2362,6 | 13661,4 | - | - | 1,7238 | - |

HFS = hochfructosehaltiger Sirup
TS = Trockensubstanz
t = Zeit (h)

1) in $t_0$ vorgelegte Menge
2) erste bis vierte Nachfüllmenge

Die in der Spalte g/Σt angegebenen Durchsätze entsprechen der Produktivität $P_t$, für deren Ermittlung folgende Beziehung gilt:

$$P_t = \frac{D_t - Do}{k} \quad [g]$$

[Do = Durchsatz in g/h zur Zeit $t_o$; $D_t$ = Durchsatz in g/h zur Zeit t; k = (ln Do - ln $D_t$)/($t_o$ - t)]

3.2 Figur 1

Verlauf der Katalysatorktivität über die Betriebsdauer des Reaktors

(1) Grundschüttung im Reaktor zum Zeitpunkt t = 0 (h)
(2) bis (5), (7) und (8) Zugabe von frischem immobilisierten Biokatalysator mit Glucoseisomerase-Aktivität am Kopf des Reaktors
(6) und (9) Zugabe von frischem immobilisierten Biokatalysator mit Glucoseisomerase-Aktivität am Kopf des Reaktors und gleichzeitige Entnahme von inaktivem Katalysator am Fuß des Reaktors
K = Katalysatoraktivität

Phase I: Anfahren des Reaktors und Aufbau des Aktivitätsprofils entlang der Reaktorsäule; (1) bis (5).
Phase II: Kontinuierliche Betriebsphase bei hohem Aktivitätsniveau; (5) bis (9).
Phase III: Herabfahren des Reaktors zur Einstellung eines geringeren Niveaus der Enzymaktivität bzw. zum Zwecke der Außerbetriebnahme; ab (9).

3.3 Figur 2

Eingesetzte Katalysatormenge und Reaktorproduktivität

(a) Produkt in kg HFS-TS
(b) Katalysator, eingesetzte Menge in g

4. Produktivität des erfindungsgemäßen Verfahrens im Vergleich zur Produktivität des konventionellen Verfahrens

Für einen konventionellen Reaktor, der mit einem Katalysatorfestbett aus immobilisierter Glucoseisomerase und ansonsten unter gleichen Bedingungen (75 °C; konstanter Isomerisierungsgrad von 46,5 %) wie das obige erfindungsgemäße Beispiel von einer Anfangsaktivität von 100 % bis zu einer Restaktivität von 20 % gefahren wird, ergibt sich eine Produktivität von 3,11 kg HFS-TS/g eingesetzten Katalysators.

Beim erfindungsgemäßen Verfahren ergibt sich für die vollkontinuierliche Betriebsphase II (hier z.B. 340-700 h), also für den Gleichgewichtszustand des Reaktorsystems, aus der in dieser Phase produzierten Menge an HFS-TS von 11,6-5,7=5,9 kg und der während dieser Phase zugeführten Katalysatormenge von 4,15-2,55=1,6 g für den Gleichgewichtszustand des Systems eine Produktivität von 3,69 kg HFS-TS/g eingesetzten Katalysators.

Aus dem Verhältnis des Produktivitätswertes des erfindungsgemäßen Verfahrens zum Produktivitätswerts des konventionellen Verfahrens ergibt sich, daß die Produktivität beim erfindungsgemäßen Verfahren um 19 % (bezogen auf die Produktivität des konventionellen Verfahrens als 100 %) höher ausfällt. Die Aktivität des Biokatalysators im erfindungsgemäßen Verfahren wird also vollständig ausgenutzt.

## Ansprüche

1. Verfahren zur kontinuierlichen Herstellung einer Lösung eines enzymatisch herstellbaren Produktes durch Umsetzung einer Substratlösung an einem immobilisierten Biokatalysator mit Enzymaktivität für die Substrat-Produkt-Umwandlung in einem Rohrreaktor, dadurch gekennzeichnet, daß man den immobilisierten Biokatalysator längs des von der Substratlösung durchströmten Rohrreaktors derart bewegt, daß längs des Rohrreaktors ein zeitlich im wesentlichen konstantes Aktivitätsprofil aufrecht erhalten wird.

2. Verfahren nach Anspruch 1, dadurch gekennzeichnet, daß das Aktivitätsprofil durch ständige Zufuhr von frischem und Abfuhr von inaktivem immobilisierten Biokatalysator aufrecht erhalten wird.

3. Verfahren nach Anspruch 1, dadurch gekennzeichnet, daß das Aktivitätsprofil durch intervallweise Zufuhr von frischem und Abfuhr von inaktivem immobilisierten Biokatalysator aufrecht erhalten wird.

4. Verfahren nach einem der vorangehenden Ansprüche, dadurch gekennzeichnet, daß man den immobilisierten Biokatalysator im Gleichstrom zur Substratlösung durch den Rohrreaktor bewegt.

5. Verfahren nach einem der vorangehenden Ansprüche, dadurch gekennzeichnet, daß man den immobilisierten Biokatalysator längs eines vertikal angeordneten Rohrreaktors, d.h. längs einer Reaktionssäule unter Ausnutzung der Gravitation bewegt.

6. Verfahren nach Anspruch 5, dadurch gekennzeichnet, daß das Aktivitätsprofil in einer Anfangsphase der Umsetzung aufgebaut wird, indem man die Umsetzung mit einer Grundschüttung aus immobilisiertem Biokatalysator in der Reaktorsäule in an sich bekannter Weise beginnt und, sobald die Enzymaktivität auf einen vorbestimmten unteren Grenzwert der Anfangsaktivität abgesunken ist, ständig oder intervallweise frischen immobilisierten Biokatalysator bis zum Erreichen einer gewünschten oder der maximalen Höhe der Schüttung zugibt.

7. Verfahren nach einem der vorangehenden Ansprüche, dadurch gekennzeichnet, daß als enzymatisch herstellbares Produkt eine Glucose und Fructose enthaltende Lösung durch Umsetzung einer Glucose enthaltenden Substratlösung an einem immobilisierten Biokatalysator mit Glucoseisomeraseaktivität hergestellt wird.

8. Verfahren nach Anspruch 7, dadurch gekennzeichnet, daß man als immobilisierten Biokatalysator eine auf porösem Siliciumdioxid oder porösem Aluminiumoxid immobilisierte Glucoseisomerase verwendet.

Fig. 1

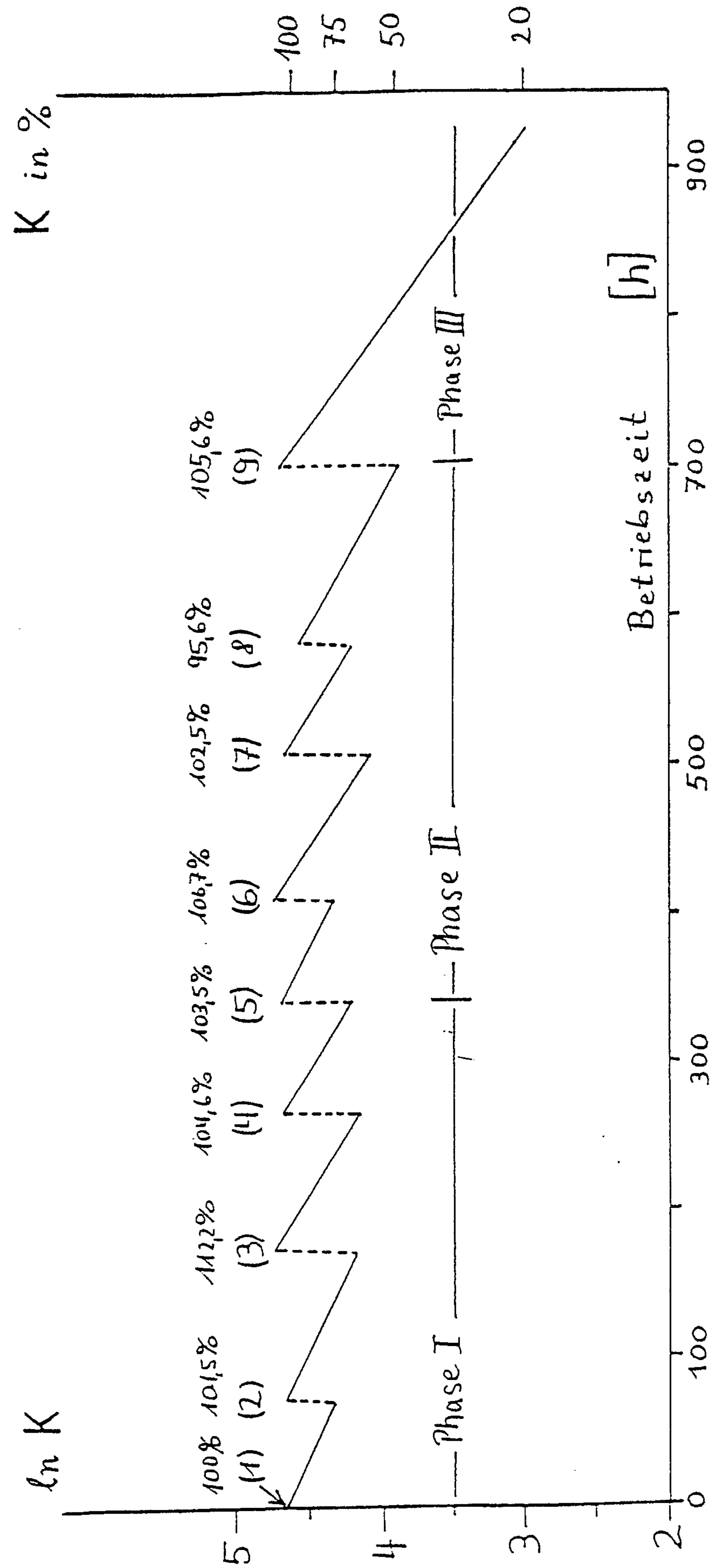
K in %
ln K
100
75
50
20
5
4
3
2
0
100
300
500
700
900
Betriebszeit [h]
Phase I
Phase II
Phase III
100% (1)
101,5% (2)
112,2% (3)
104,6% (4)
103,5% (5)
106,7% (6)
102,5% (7)
95,6% (8)
105,6% (9)

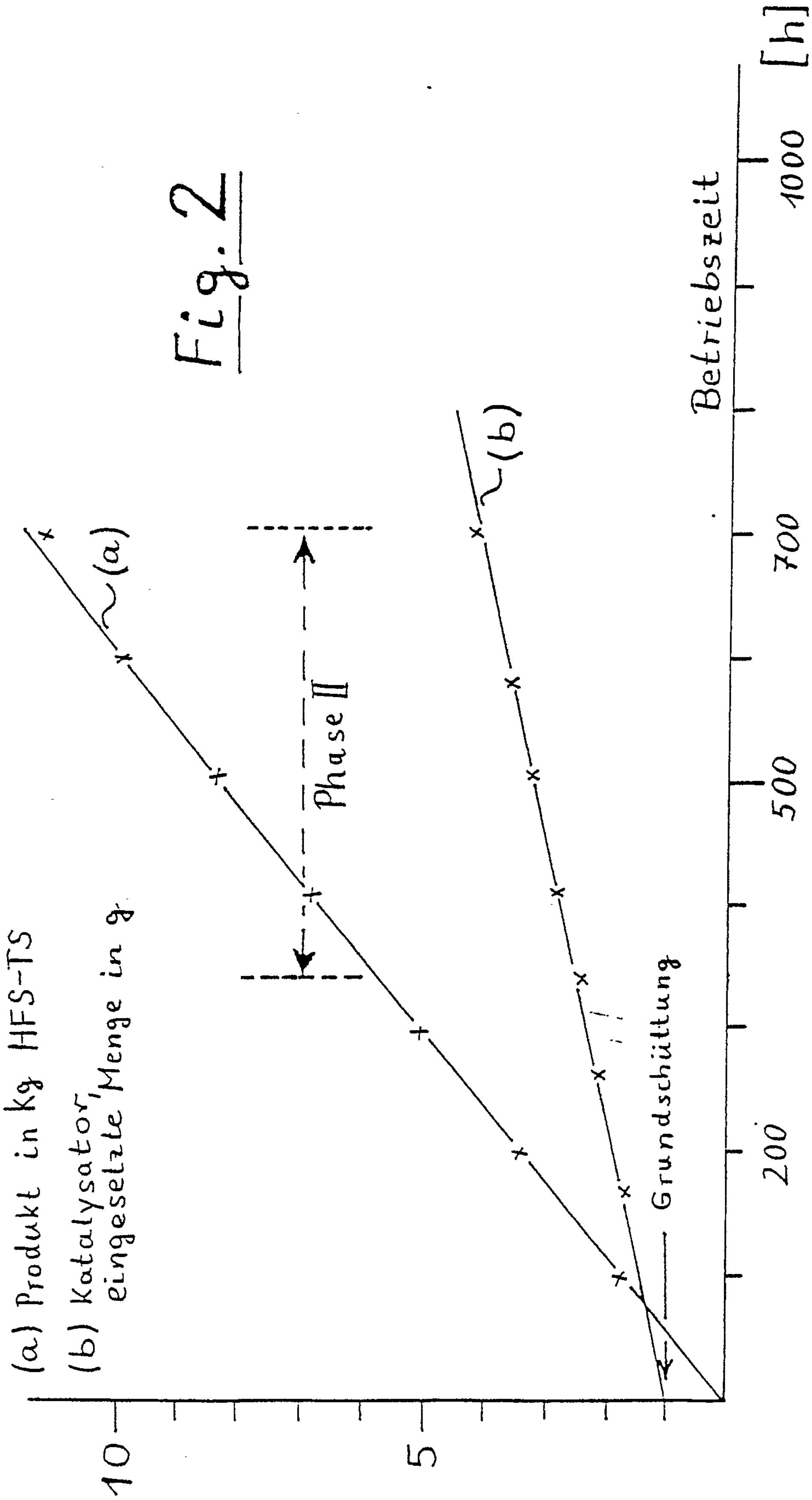
Fig. 2
(a) Produkt in kg HFS-TS
(b) Katalysator, eingesetzte Menge in g
10
5
Phase II
(a)
(b)
Grundschüttung
200
500
700
1000
Betriebszeit
[h]

Europäisches Patentamt

# EUROPÄISCHER RECHERCHENBERICHT

Nummer der Anmeldung

**EP 90 11 5010**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | US-A-4 158 609 (H. MULLER)<br>* Fig.; Anspr.; Spalte 2, Zeile 15 - Spalte 4, Zeile 38 *<br>– – – | 1-8 | C 12 M 1/40 |
| X | US-A-4 442 216 (D.G. HARVEY et al.)<br>* Anspr.; Fig. *<br>– – – | 1-4,6 | |
| X | FR-A-2 400 386 (STAMICARBON B.V.)<br>* Fig.; Seite 1, Zeile 13 - Seite 2; Seite 5, Seite 4, Zeilen 15-39; Seite 5; Seite 6, Zeile 1-21; Seite 8, Zeilen 2-23; Seite 11; Seite 12, Zeilen 1-14 *<br>– – – | 1-3,5-8 | |
| X | FR-A-2 347 091 (MILES LABORATORIES INC.)<br>* Seite 1, Zeile 21 - Seite 2, Zeile 26; Seite 4, Zeile 23 - Seite 7, Zeile 26; Fig.; Anspr. *<br>– – – | 1-8 | |
| X | EP-A-0 042 306 (EXXON RESEARCH AND ENGINEERING CO.)<br>* Fig.; Anspr.; Seite 7, Zeile 25 - Seite 8, Zeile 23 *<br>– – – | 1,2,4-6 | |
| A | | 7,8 | |
| X | CHEMICAL ABSTRACTS, Band 94, Nr. 13, März 1981, Seite 581, Zusammenfassung Nr. 101348r, Columbus, Ohio, US; & JP-A-80 42 837 (DENKI KAGAKU KOGYO K.K. DENKA ENGINEERING CO., LTD) 01-11-1980<br>* Zusammenfassung *<br>– – – | 1,3,5-8 | **RECHERCHIERTE SACHGEBIETE (Int. Cl.5)**<br>C 12 M |
| X | EP-A-0 149 151 (KRAUSS-MAFFEI AG)<br>* Figuren 1-4; Seite 2, Zeilen 14-18; Anspr. *<br>– – – – – | 1,4,5 | |

Der vorliegende Recherchenbericht wurde für alle Patentansprüche erstellt

| Recherchenort | Abschlußdatum der Recherche | Prüfer |
|---|---|---|
| Den Haag | 27 November 90 | COUCKE A.O.M. |

**KATEGORIE DER GENANNTEN DOKUMENTE**

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze

E: älteres Patentdokument, das jedoch erst am oder nach dem Anmeldedatum veröffentlicht worden ist
D: in der Anmeldung angeführtes Dokument
L: aus anderen Gründen angeführtes Dokument

&: Mitglied der gleichen Patentfamilie, übereinstimmendes Dokument